# EUROPEAN PATENT APPLICATION

(11) **EP 2 022 339 A1**
(43) Date of publication of application: **11.02.2009**
(21) Application number: 07743580.8
(22) Date of filing: 17.05.2007
(51) Int. Cl.: A23K 1/18, A01K 67/033, A01K 67/04, A23K 1/16, C12N 1/00, C12N 15/09

(54) **ARTIFICIAL DIET FOR LEPIDOPTERAN AND METHOD FOR PRODUCING THE SAME, LEPIDOPTERAN AND METHOD FOR PRODUCING THE SAME, AND BIOMATERIAL**

(30) Priority: 19.05.2006 JP 2006140747
(71) Applicant: Yamaguchi University, Yamaguchi 753-8511 (JP); NATIONAL INSTITUTE OF AGROBIOLOGICAL SCIENCES, Tsukuba-shi, Ibaraki 305-8602 (JP); Taiyo Nippon Sanso Corporation, Tokyo 142-8558 (JP)
(72) Inventor: KOBAYASHI, Jun, Yamaguchi-shi, Yamaguchi 753-8511 (JP); NAKAMURA, Masatoshi, Tsukuba-shi, Ibaraki 305-8602 (JP); YOKOYAMA, Jun, Tokyo 142-8558 (JP)
(74) Representative: Waldren, Robin Michael
(86) International application number: PCT/JP2007/060145
(87) International publication number: WO 2007/135953

(57) **Abstract**

An artificial diet for lepidopteran insects of the present invention, including: a protein; and a carbohydrate, wherein a content of a stable isotope is equal to or greater than 50 atom%. A method for producing an artificial diet for lepidopteran insects, including: a defatting treatment step of subjecting a microorganism to a defatting treatment; a hydrothermal solution extraction treatment step of subjecting the microorganism to an extraction treatment using a hydrothermal solution; and a mixing step of mixing a carbohydrate and a protein originated from the microorganism that is subjected to the defatting treatment step and the hydrothermal solution extraction treatment step.

## Description

### TECHNICAL FIELD

The present invention relates to an artificial diet for lepidopteran insects and a production method thereof, a lepidopteran insect and a production method thereof, and a biological material.
Priority is claimed on Japanese Patent Application No. 2006-140747, filed May 19, 2006, the content of which is incorporated herein by reference.

### BACKGROUND ART

In recent years, whole genome sequences of various organisms have been determined. In addition, based on the information, intensive studies have been carried out for comprehensively determining the three dimensional structures of proteins encoded in the genomes.
Nuclear magnetic resonance spectroscopy (hereinafter referred to as "NMR") is one of the available approaches for determining the three dimensional structures of proteins. When measuring a protein by NMR, it is usually necessary to introduce a stable isotope such as ¹³C, ¹⁵N, and ²H into the target protein.
In addition, owing to the recent developments of the analytical techniques in the field of mass spectrometry and the wide spread of general purpose mass spectrometers, qualitative analysis of biological materials as well as quantitative analysis thereof have widely been carried out, not only at species level but also at individual level. Also in this technical field, especially in the quantitative analysis, it is important to introduce a stable isotope into the targeted biological material.
It should be noted that the term "biological material" used here is a generic term that includes proteins, carbohydrates, and lipids that are produced by organisms, as well as other materials of biological origin.

As a method for introducing stable isotopes in proteins for the sake of analyzing proteins by NMR, for example, the following technique to synthesize a desired protein labeled with a stable isotope has been established. That is, genetically engineered *Escherichia coli* cells or yeast cells are cultured in a medium containing a carbon source (such as D-glucose-¹³C₆ and methanol-¹³C), a nitrogen source (such as ammonium chloride-¹⁵N and ammonium sulfate-¹⁵N), or water (such as D₂O and H₂¹⁸O) labeled with a stable isotope so that the target protein is overexpressed extracellularly or intracellularly.

However, in the abovementioned method for synthesizing proteins labeled with a stable isotope using genetically engineered *E. coli* cells, post-translational modifications that are extremely important in the function of proteins of higher organisms, such as glycosylation, phosphorylation, methylation, and acetylation, do not take place. Accordingly, there are many cases where active proteins cannot be obtained.
Moreover, when using genetically engineered yeast cells, although post-translational modifications do take place, the modified sites and/or the types of sugar chains were different from those in higher organisms causing various problems. Furthermore, the membrane proteins that are said to have important functions *in vivo* had also been extremely difficult to synthesize with the abovementioned conventional technique.

On the other hand, in recent years, as a method for synthesizing various proteins including the proteins modified post-translationally, such as glycoproteins and phosphoproteins, as well as the membrane proteins, a technique for synthesizing a large amount of desired proteins has been established by first manipulating genes in a strain of baculovirus that specifically infects insects and then infecting a lepidopteran insect such as a silkworm with the strain of genetically engineered baculovirus (for example, refer to Patent Documents 1 to 3). With this method, it is possible to synthesize proteins that are post-translationally modified in a manner widely shared by higher organisms as well as membrane proteins at a high success rate which has been impossible to accomplish with the abovementioned method using genetically engineered *E. coli* and yeast cells. In addition, this system of producing proteins using lepidopteran insects infected with the genetically engineered baculovirus or transgenic lepidopteran insects is already at a stage for practical use, and thus it has been commercialized in the field of drug production for animals, contract manufacturing of proteins for research, and the like.
It should be noted here that the term "lepidopteran insect" used in the present invention is a generic term that includes native lepidopteran insects, lepidopteran insects infected with a baculovirus, and transgenic lepidopteran insects.

When analyzing the biological materials originated from higher organisms such as lepidopteran insects, it is necessary to introduce a stable isotope into the biological materials as mentioned earlier. Accordingly, it is necessary to first introduce a stable isotope in higher organisms in high concentrations.
As a method for introducing a stable isotope in this process, a method is proposed in which a desired organism is fed with food labeled with a stable isotope (for example, refer to Patent Document 4). However, many higher organisms including lepidopteran insects have a food preference unlike microorganisms, although depending on the type thereof. Accordingly, foods which do not satisfy their preference cannot be used even if the foods contain sufficient nutrition. In other words, the foods that satisfy the preference of higher organisms need to be labeled with a stable isotope.
Patent Document 4 discloses a method in which plants favored by lepidopteran insects are grown with adequately regulated ¹³CO₂ and nutrients labeled with ¹⁵N in a completely sealed chamber. However, with such a method, facilities for growing the plants favored by the insects will be expensive, time of no less than 1 month will be required for the growth of the plants, and the utilization efficiency of the materials for stable isotope labeling will be extremely low. Due to the presence of such problems, the method described in Patent Document 4 is not realistic from an economic perspective.
[Patent Document 1] Japanese Unexamined Patent Application, First Publication No. Sho 61-9288
[Patent Document 2] Japanese Unexamined Patent Application, First Publication No. Sho 61-9297
[Patent Document 3] Japanese Unexamined Patent Application, First Publication No. Sho 62-208276
[Patent Document 4] Japanese Unexamined Patent Application, First Publication No. 2006-53100

### DISCLOSURE OF THE INVENTION

### PROBLEMS TO BE SOLVED BY THE INVENTION

In view of the abovementioned problems associated with the conventional technique, an object of the present invention is to provide an artificial diet for lepidopteran insects which is highly labeled with a stable isotope and is also capable of satisfactorily growing lepidopteran insects and a production method thereof, a lepidopteran insect highly labeled with a stable isotope and a production method thereof, and a biological material highly labeled with a stable isotope.

### MEANS FOR SOLVING THE PROBLEMS

In order to solve such problems, the present invention is composed of the following aspects. A first aspect of the present invention is an artificial diet for lepidopteran insects containing a protein and a carbohydrate in which the content of a stable isotope is equal to or greater than 50 atom%.
Note that the content of a stable isotope in the present invention refers to the ratio of different stable isotopes of the same element in terms of the amount thereof (atom%).
In addition, it should be noted that in the calculation of the content of a stable isotope, only the components in the artificial diet for lepidopteran insects that can be broken down and be absorbed by lepidopteran insects are taken into account. In other words, in the artificial diet for lepidopteran insects, the components that cannot be broken down and/or be absorbed by lepidopteran insects are omitted from the calculation.

In the artificial diet for lepidopteran insects according to the present invention, it is preferable that the aforementioned protein be originated from microorganisms. Also, it is preferable that the microorganisms be one, or two or more microorganisms selected from the group consisting of algae, bacteria including photosynthetic bacteria, fungi, and yeasts. In addition, it is preferable that the microorganism be subjected to a defatting treatment as well as an extraction treatment using a hydrothermal solution. Moreover, it is preferable that the artificial diet further contains an inorganic salt and a vitamin. Furthermore, it is preferable that the stable isotope be one, or two or more stable isotopes selected from the group consisting of ¹³C, ¹⁵N, ²H, ¹⁷O, ¹⁸O, and ³³S.

A second aspect of the present invention is a method for producing an artificial diet for lepidopteran insects including a defatting treatment step of subjecting microbial cells to a defatting treatment; a hydrothermal solution extraction treatment step of subjecting microbial cells to an extraction treatment using a hydrothermal solution; and a mixing step of mixing a carbohydrate and a protein originated from the microbial cells that are subjected to the defatting treatment step and the hydrothermal solution extraction treatment step.

A third aspect of the present invention is a lepidopteran insect in which the content of a stable isotope is equal to or greater than 50 atom%.
The lepidopteran insect of the present invention is preferably an insect that has been grown by feeding with the artificial diet for lepidopteran insects according to the present invention. In addition, the lepidopteran insect of the present invention is preferably an insect that is infected with a genetically engineered baculovirus.

A fourth aspect of the present invention is a method for producing a lepidopteran insect including a step for of growing the lepidopteran insect by feeding with the artificial diet for lepidopteran insects according to the present invention.
In the method of the present invention for producing a lepidopteran insect, it is preferable that the method further includes a step of subjecting the lepidopteran insect to an infection treatment with a genetically engineered baculovirus.

A fifth aspect of the present invention is a biological material produced by the lepidopteran insect of the present invention.

### EFFECTS OF THE INVENTION

By using the artificial diet for lepidopteran insects according to the present invention, it will be possible to obtain a lepidopteran insect that is highly labeled with a stable isotope.
In addition, by using this lepidopteran insect, it will be possible to produce biological materials, especially post-translationally modified proteins and membrane proteins, that are highly labeled with a stable isotope which have been difficult to produce with the conventional techniques.
Moreover, since the above biological materials are highly labeled with a stable isotope, their qualitative analysis as well as their quantitative analysis can be carried out by mass spectrometry.
Furthermore, since the above proteins are highly labeled with a stable isotope, the three dimensional structures of proteins, post-translationally modified proteins, and membrane proteins can be analyzed by NMR.

### BEST MODE FOR CARRYING OUT THE INVENTION

One embodiment of the present invention will be described in detail below.

### (Artificial diet for lepidopteran insects)

The artificial diet for lepidopteran insects according to the present invention contains a protein and a carbohydrate. In addition, the content of stable isotope in this artificial diet for lepidopteran insects is equal to or greater than 50 atom%, preferably equal to or greater than 70 atom%, and more preferably equal to or greater than 90 atom%.
If the content of stable isotope is equal to or greater than 50 atom%, it will be possible to obtain a lepidopteran insect that is highly labeled with a stable isotope when the lepidopteran insect has been grown using the artificial diet for lepidopteran insects according to the present invention. In addition, since the biological material extracted from such a lepidopteran insect is highly labeled with a stable isotope, high sensitivity can be achieved in the NMR measurements. Accordingly, the biological material can be analyzed with precision. On the other hand, when the content of stable isotope is less than 50 atom%, it will be impossible to obtain a lepidopteran insect and a biological material that are highly labeled with a stable isotope. Precise analysis cannot be conducted by NMR measurements using such biological materials due to insufficient sensitivity.
When the content of stable isotope is equal to or greater than 70 atom%, not only the SN ratio (ratio between signal and noise) improves, but also the time for NMR measurements can be shortened. In addition, when the content of stable isotope is equal to or greater than 90 atom%, multidimensional analysis of polynuclear species will be possible which enables the analysis of biological materials including more complex proteins.

In the present invention, the upper limit for the content of stable isotope is 100 atom%. When the content of stable isotope is 100 atom%, sensitivity in the NMR measurements will be extremely high. However, it should be noted that the content of stable isotope of 100 atom% is impossible to achieve with the currently available technique, and thus the actual upper limit should be the value close to 100 atom% but less than 100 atom%. Accordingly, the range for the content of stable isotope is equal to or greater than 50 atom% and less than 100 atom%, preferably equal to or greater than 70 atom% and less than 100 atom%, and more preferably equal to or greater than 90 atom% and less than 100 atom%.
Note that also in mass spectrometry, a high content of stable isotope in the biological material leads to an easy detection thereof, and thus its precise quantitative analysis will be possible.

The type of protein to be used in the artificial diet for lepidopteran insects according to the present invention is not particularly limited as long as it is labeled with a stable isotope. The content of stable isotope in this protein may be adequately adjusted so that the content of stable isotope in the artificial diet for lepidopteran insects will be equal to or greater than 50 atom% while taking the amount of components other than the protein into consideration.
The above protein may originate from one, or two or more of an animal, a plant, and a microorganism. However, it is preferable that the protein originates from a microorganism due to the easy introduction of a stable isotope therein. For such a microorganism, algae, bacteria including photosynthetic bacteria, fungi, and yeasts may be exemplified. Of these, algae and photosynthetic bacteria are preferable since they are capable of fixing ¹³CO₂ through photosynthesis. It should be noted here that as mentioned earlier while describing the conventional technique, there are problems in using animals since they have preferences towards some specific foods, and there are problems in using plants since the utilization efficiency of the materials for stable isotope labeling is extremely low. Accordingly, it is necessary to solve these problems in advance when using the proteins originated from animals and plants.

Since various methods have been developed for introducing a stable isotope in microorganisms and have been well known already, an adequate method may be selected from them depending on the purpose. For example, a method can be selected in which microbial cells are cultured in a medium containing a carbon source, a nitrogen source, or water which is labeled with a stable isotope.

Since microorganisms contain a repellent (for example, an oxidized fatty acid) that repels lepidopteran insects from ingesting those or a growth inhibitor, it is preferable that microorganisms be used after these substances are removed from them. As the method for removing these substances, a defatting treatment and an extraction treatment with a hydrothermal solution are preferable.

The defatting treatment is a process in which microorganisms are heated in an organic solvent for extraction. Due to this process, it is possible to remove hydrophobic components in the repellents and growth inhibitors. Examples of the above organic solvent include methanol, chloroform, hexane, diethyl ether, and methylene chloride. These organic solvents may be used alone or two or more kinds thereof may be combined for use. The temperature of organic solvents may be from room temperature up to a temperature where the solvents can be refluxed, and the temperature is determined depending on the type of organic solvent to be used. The time for conducting a defatting treatment differs depending on the type of microorganisms and the type of organic solvent to be used, although 1 to 10 hours may be exemplified. Note that the defatting treatment may be conducted more than once and may be conducted several times if necessary.

The extraction treatment using a hydrothermal solution is a process in which microorganisms are heated in a hydrothermal solution for extraction. Due to this process, it is possible to remove hydrophilic components in the repellents and growth inhibitors. If necessary, a buffering agent, a surfactant, an inorganic salt, or the like can be added to the abovementioned hydrothermal solution where appropriate. The temperature of the hydrothermal solution may be from room temperature up to a temperature where the solution can be refluxed, and specifically, the temperature of 90°C to 140°C may be exemplified.
The time for conducting an extraction treatment using a hydrothermal solution differs depending on the type of microorganisms to be used, although 0.5 to 5 hours may be exemplified. Note that the extraction treatment using a hydrothermal solution may be conducted more than once and may be conducted several times if necessary.

By removing the repellents and growth inhibitors contained in the microorganisms as described above, it will be possible to produce an artificial diet for lepidopteran insects which satisfies their preference and supports their growth excellently.
Note that the order in which the defatting treatment and the extraction treatment with a hydrothermal solution are conducted is not particularly limited.

The type of carbohydrate to be used in the artificial diet for lepidopteran insects according to the present invention is not particularly limited as long as it is labeled with a stable isotope and is assimilable by lepidopteran insects.
Although starch, agar, and cellulose which are used as the components of the artificial diet for lepidopteran insects improve the feeding of lepidopteran insects, many of the lepidopteran insects do not have a digestive enzyme to breakdown these substances. Accordingly, these substances are not suited as a carbohydrate in the present invention.
The content of stable isotope in this carbohydrate may be adequately adjusted so that the content of stable isotope in the artificial diet for lepidopteran insects will be equal to or greater than 50 atom% while taking the amount of components other than the carbohydrate into consideration.
The abovementioned carbohydrates will be available by purchasing the commercial products.

Apart from the abovementioned protein and carbohydrate, a lipid, an organic acid, an inorganic salt, a vitamin, a powder originated from plants, or the like which is a component acceptable as an artificial diet for lepidopteran insects can be added to the artificial diet for lepidopteran insects according to the present invention where appropriate. These components are preferably labeled with a stable isotope in view of enhancing the content of stable isotope in the artificial diet for lepidopteran insects.

The stable isotope used in the artificial diet for lepidopteran insects according to the present invention is preferably one, or two or more stable isotopes selected from the group consisting of ¹³C, ¹⁵N, ²H, ¹⁷O, ¹⁸O, and ³³S. These stable isotopes are useful in the analysis by mass spectrometry and NMR since they are the elements that constitute biological materials such as proteins. In addition, it is more preferable that the aforementioned stable isotope be one, or two or more stable isotopes selected from the group consisting of ¹³C, ¹⁵N, ²H, and ¹⁷O. ¹³C, ¹⁵N, and ¹⁷O are nuclear species that are detectable in the NMR measurements and a lot of information on the structures of biological materials, such as proteins, can be brought about. In addition, ²H can eliminate the peak signal originated from ¹H by substituting this ¹H. As a result, NMR measurement data can be simplified and thereby making the analysis thereof easier.

### (Production method of artificial diet for lepidopteran insects)

The method of the present invention for producing an artificial diet for lepidopteran insects includes a defatting treatment step of subjecting microorganisms to a defatting treatment; a hydrothermal solution extraction treatment step of subjecting microorganisms to an extraction treatment using a hydrothermal solution; and a mixing step of mixing a carbohydrate and a protein originated from the microorganisms that are subjected to the defatting treatment step and the hydrothermal solution extraction treatment step.

As described earlier, since microorganisms contain a repellent (for example, an oxidized fatty acid) that repels lepidopteran insects from ingesting those or a growth inhibitor, it is preferable that microorganisms be used after the repellents or the growth inhibitors are removed from them by the abovementioned defatting treatment and hydrothermal solution extraction treatment. Due to these treatments, it will be possible to produce an artificial diet for lepidopteran insects which satisfies their preference and supports their growth excellently. The operating conditions in which the defatting treatment and the hydrothermal solution extraction treatment are the same as those described above.
Methods for conducting the mixing step are not particularly limited and known methods can be selected. For example, the artificial diet for lepidopteran insects can be produced by mixing the abovementioned protein and carbohydrate originated from microorganisms as well as other components by a known method.
Note that it is preferable to adjust the content of stable isotope in a protein, a carbohydrate, and other components so that the content of stable isotope in the artificial diet for lepidopteran insects obtained after the mixing step will be equal to or greater than 50 atom%.

### (Lepidopteran insects and transgenic lepidopteran insects)

The lepidopteran insects of the present invention are characterized by having a content of stable isotope of 50 atom% or more. In addition, the content of stable isotope in the lepidopteran insects is preferably equal to or greater than 70 atom% and more preferably equal to or greater than 90 atom%.
Since the biological material extracted from the lepidopteran insect of the present invention is highly labeled with a stable isotope when the content of stable isotope is equal to or greater than 50 atom%, high sensitivity can be achieved in the NMR measurements. Accordingly, the biological material can be analyzed with precision. On the other hand, when the content of stable isotope is less than 50 atom%, it will be impossible to obtain a biological material that is highly labeled with a stable isotope. Precise analysis cannot be conducted by NMR measurements using such biological materials due to insufficient sensitivity.
When the content of stable isotope is equal to or greater than 70 atom%, not only the SN ratio improves, but also the time for NMR measurements can be shortened. In addition, when the content of stable isotope is equal to or greater than 90 atom%, multidimensional analysis of polynuclear species will be possible which enables the analysis of biological materials including more complex proteins.

In the present invention, the upper limit for the content of stable isotope is 100 atom%. When the content of stable isotope is 100 atom%, sensitivity in the NMR measurements will be extremely high. However, it should be noted that the content of stable isotope of 100 atom% is impossible to achieve with the currently available technique, and thus the actual upper limit should be the value close to 100 atom% but less than 100 atom%. Accordingly, the range for the content of stable isotope is equal to or greater than 50 atom% and less than 100 atom%, preferably equal to or greater than 70 atom% and less than 100 atom%, and more preferably equal to or greater than 90 atom% and less than 100 atom%.
Note that also in mass spectrometry, a high content of stable isotope in the biological material leads to an easy detection thereof, and thus its precise quantitative analysis will be possible.

The type of lepidopteran insects of the present invention is not particularly limited as long as it can be grown with the artificial diet for lepidopteran insects according to the present invention. For example, a polyphagous lepidopteran insect that does not have a specifically favorite plant and appears relatively polyphagous can be suitably used since it can be grown satisfactorily with the artificial diet for lepidopteran insects. Examples of such polyphagous lepidopteran insects include *Samia cynthia ricini*, an armyworm, *Hyphantria cunea, Samia cynthia pryeri*, and *Caligula japonica*.

In addition, mainly in sericulture, the lepidopteran insects that have been bred by artificial crossing for a long time in order to reduce their preference and make them polyphagous can also be used suitably. Examples of such lepidopteran insects bred to become polyphagous include the polyphagous silkworm races "Asagiri", "Shin-Asagiri", "Habataki", and "Honobono" bred at the National Institute of Agrobiological Sciences that used to be known as the National Institute of Sericultural and Entomological Science.
By growing these lepidopteran insects by feeding with the artificial diet for lepidopteran insects according to the present invention, it will be possible to obtain a lepidopteran insect that is highly labeled with a stable isotope.

The lepidopteran insect of the present invention is preferably infected with a genetically engineered baculovirus. Due to this infection process, a foreign gene encoding a desired protein can be introduced in a lepidopteran insect. As a result, it will be possible to produce a large amount of desired proteins (including membrane proteins) that are highly labeled with a stable isotope and are also post-translationally modified in a manner widely shared by higher organisms in the body fluids and tissues of lepidopteran insects in addition to the biological materials that are originally produced by the lepidopteran insects.

In addition, a transgenic lepidopteran insect in which a foreign gene is inserted in its genome can be used as the lepidopteran insect of the present invention. The transgenic lepidopteran insect refers to a genetically engineered lepidopteran insect produced by employing a technique in which a foreign gene is inserted in the genome of the lepidopteran insect using a special vector known as a transposon found from the virus infecting the insect. By using this transgenic lepidopteran insect, it will be possible to obtain effects that are the same as those achieved with the lepidopteran insects infected with a genetically engineered baculovirus.

### (Production method of lepidopteran insects)

The method of the present invention for producing a lepidopteran insect includes a step of growing a lepidopteran insect by feeding with the artificial diet for lepidopteran insects according to the present invention. In terms of the method for feeding the insect, the artificial diet for lepidopteran insects according to the present invention may be fed to the lepidopteran insects from their hatching stage or may be fed to the lepidopteran insects in the midst of their growing stage by replacing the artificial diet that does not contain a stable isotope. Since lepidopteran insects show a large appetite especially from the fifth instar to the last instar, it is preferable to feed the artificial diet for lepidopteran insects according to the present invention during these growth stages from an economic perspective.

Methods for growing the insects are not particularly limited and known methods can be employed. However, when using a nuclear species such as ²H, ¹⁷O, and ¹⁸O which may interchange with the elements in the atmospheric moisture as a stable isotope, it is preferable to grow the insects in a sealed space by using a sealed box. When these nuclear species are not used, there is no particular need to grow the insects in a sealed space.
By using the method of the present invention for producing lepidopteran insects as described so far, it will be possible to obtain a lepidopteran insect that is highly labeled with a stable isotope.

In the method of the present invention for producing a lepidopteran insect, it is preferable that the method further includes a step of subjecting the lepidopteran insect to an infection treatment with a genetically engineered baculovirus. Specifically, a method can be employed in which a gene encoding a desired protein is first introduced in a baculovirus using a genetic engineering approach and then infecting a lepidopteran insect with the obtained genetically engineered baculovirus by a known method. Due to this step, it will be possible to produce a large amount of desired proteins (including membrane proteins) that are highly labeled with a stable isotope and are also post-translationally modified in a manner widely shared by higher organisms in the body fluids and tissues of lepidopteran insects in addition to the biological materials that are originally produced by the lepidopteran insects.

It should be noted that when using a transgenic lepidopteran insect as a lepidopteran insect, the step of growing the insect by feeding with the artificial diet for lepidopteran insects according to the present invention alone is enough to achieve the same effects as those achieved in the method of the present invention for producing a lepidopteran insect which further includes the step of subjecting the insect to an infection treatment with a genetically engineered baculovirus.

### (Biological materials)

The biological materials in the present invention include proteins, carbohydrates, and lipids that are produced by the lepidopteran insects of the present invention, as well as other materials originated from lepidopteran insects. Since these biological materials are highly labeled with a stable isotope, they are useful in the analysis by NMR, mass spectrometry, and the like.
In order to obtain the biological materials of the present invention, a lepidopteran insect or transgenic lepidopteran insect of the present invention is first produced, and then the biological materials produced by the insect are extracted by a known extraction method. Specifically, a method can be employed in which the lepidopteran insect or transgenic lepidopteran insect is first crushed or ground and the resultant is then subjected to a centrifugal separation. The biological materials of the present invention can be obtained by purifying the targeted biological material using an adequate purification process from the extracted solution obtained as described above.

### EXAMPLES

The present invention will be described in more detail below using Examples. However, the present invention is not limited to the following Examples.

### (Example 1)

As a protein added to the artificial diet for lepidopteran insects according to the present invention, a protein originating from *Spirulina,* a type of algae, was used. *Spirulina* cells were cultured in a culture medium having a composition shown in Table 1 which contained ¹⁵N labeled sodium nitrate for 14 days. After the cultivation, the *Spirulina* cells in the culture medium were collected using a continuous centrifugal separator. The collected cells were first washed with ion exchanged water and then frozen with liquid nitrogen, and thereafter freeze dried directly in a freeze dryer.

**[Table 1]**

| Composition of culture medium for labeling *Spirulina* with ¹⁵N | | | |
|---|---|---|---|
| Compound | Weight | | |
| | g | *A5 solution | |
| NaHCO₃ | 16.8 | H₃BO₃ | 286 mg |
| K₂HPO₄ | 0.5 | MnSO₄·7H₂O | 250 mg |
| Na¹⁵NO₃ | 2.5 | ZnSO₄·7H₂O | 22.2 mg |
| K₂SO₄ | 1 | CuSO₄·SH₂O | 7.9 mg |
| NaCl | 1 | Na₂MoO₄·2H₂O | 2.1 mg |
| MgSO₄·7H₂O | 0.2 | Distilled Water | 100 ml |
| CaCl₂·2H₂O | 0.04 | | |
| FeSO₄·7H₂O | 0.01 | | |
| Na₂EDTA | 0.08 | | |
| Distilled Water | Fill up to 1 L | | |
| A5 solution* | 0.1 ml | | |

About 25 g of the *Spirulina* cells obtained by drying was finely ground and all of the resultant was placed in a 2L eggplant shaped flask. Subsequently, a defatting treatment was carried out by first adding 1L of a mixed solvent of methanol and chloroform (2: 1) to the flask, and then subjecting the mixture to a reflux/extraction by heating for 3 hours due to the heating of the flask to 90°C using an oil bath. Thereafter, the mixture was cooled down to room temperature and then filtered using a Teflon filter. About 20 g of defatted *Spirulina* cells was obtained by drying the matter obtained by the filtration process in a vacuum dryer.

A hydrothermal solution extraction treatment was carried out by placing the dried and defatted *Spirulina* cells in a 2L eggplant shaped flask, adding 1 L of ion exchanged water thereto, and then subjecting the resultant to a hydrothermal extraction process while refluxing for 1 hour due to the heating of the flask to 120°C using an oil bath. After 1 hour of the hydrothermal extraction process, the resultant was cooled down to room temperature and the contents therein were collected by centrifugal separation at 8,000 rpm for 30 minutes using a centrifugal separator. Supernatant was removed by decantation and by the use of a pipette and the precipitates were transferred back to the 2L eggplant shaped flask. A hydrothermal solution extraction treatment was then carried out again by subjecting the precipitates to a hydrothermal extraction process while refluxing. After repeating the abovementioned hydrothermal solution extraction treatment for another two rounds, the resultant was washed twice with cold ion exchanged water and twice with 70% ethanol. About 11 g of the extracted fraction of crude *Spirulina* proteins labeled with ¹⁵N was obtained by washing the precipitates obtained above with diethyl ether and then completely drying them.

The artificial diet for lepidopteran insects containing the abovementioned extracted fraction of crude *Spirulina* proteins labeled with ¹⁵N was mixed so as to achieve the composition shown in Table 2 as K25, uniformly kneaded while being heated, and was then steamed and formed into a rectangular shape. On the other hand, the artificial diets for lepidopteran insects containing only a defatted soybean powder or this powder as well as a mulberry leaf powder as protein sources instead of the extracted fraction of crude *Spirulina* proteins labeled with ¹⁵N were produced in a similar manner so as to achieve the compositions shown as KS40 and KM25 in Table 2, and they were used as controls.
In the artificial diet K25 for lepidopteran insects, most nitrogen in the diet components was replaced with ¹⁵N and the content of stable isotope (¹⁵N) in the artificial diet for lepidopteran insects was equal to or greater than 90 atom%.

**[Table 2]**

| Composition of artificial diet for lepidopteran insects | | | |
|---|---|---|---|
| | ¹⁵N labeled artificial diet | Semisynthetic diet | Diet containing mulberry |
| | K25 | KS40 | KM25 |
| ¹⁵N labeled crude protein | 11.00 g | | |
| Mulberry leaf powder | | | 12.50 g |
| Defatted soybean powder | | 20.00 g | 12.00 g |
| Soybean oil | 1.32 g | 1.50 g | 1.50 g |
| Glucose | 4.40 g | | |
| Sucrose | | 5.00 g | 5.00 g |
| Starch | 4.40 g | 5.00 g | 4.00 g |
| Agar | 4.40 g | 5.00 g | 5.00 g |
| Phytosterol | 0.13 g | 0.15 g | 0.15 g |
| Inorganic salt mixture | 2.64 g | 3.00 g | 3.00 g |
| Mixture containing vitamins | 0.44 g | 0.50 g | 0.50 g |
| Ascorbic acid | 0.88 g | 1.00 g | 1.00 g |
| Citric acid | 1.76 g | 2.00 g | 2.00 g |
| Cellulose | 16.18 g | 6.75 g | 3.25 g |
| Sorbic acid | 0.09 g | 0.10 g | 0.10 g |
| Powder (in total) | 47.64 g | 50.00 g | 50.00 g |
| Propionic acid | 0.33 ml (≈g) | 0.38 ml (≈g) | 0.38 ml (≈g) |
| Water | 131.67 ml (≈g) | 149.63 ml (≈g) | 149.63 ml (≈g) |
| Total | 179.64 g | 200.00 g | 200.00 g |

In addition, compositions of the inorganic salt mixture and the mixture containing vitamins in Table 2 are shown in Table 3 and 4, respectively.

**[Table 3]**

| Inorganic salt mixture | |
|---|---|
| Substance | |
| K₂HPO₄ | 150 g |
| KCl | 48 g |
| MgHPO₄/3H₂O | 52 g |
| CaCO₃ | 84 g |
| FePO₄/4H₂O | 7 g |
| Total | 341 g |

**[Tale 4]**

| Mixture containing vitamins | |
|---|---|
| Substance | Content (g) |
| Biotin | 0.02 |
| Folic acid | 0.02 |
| Thiamine - HCl | 0.20 |
| Riboflavin | 0.20 |
| Pyridoxine - HCl | 0.30 |
| Nicotinic acid | 1.00 |
| Ca - panthothenate | 1.50 |
| Choline - Cl | 15.00 |
| Inositol | 20.00 |
| Chloramphenicol | 1.50 |
| Cellulose powder | 60.26 |
| Total | 100.00 |

The artificial diet K25 for lepidopteran insects was fed to a fifth instar larva of *Samia cynthia ricini*, a type of a lepidopteran insect. In addition, each of the artificial diets KS40 and KM25 for lepidopteran insects which were used as controls were also fed to the different fifth instar larvae of *Samia cynthia ricini* in a similar manner. The fifth instar larvae of *Samia cynthia ricini* were fed with the artificial diet K25, KS40, or KM25 for lepidopteran insects in a manner described above for 12 days and were grown until they pupated. It should be noted that the same amount of artificial diet for lepidopteran insects was fed to each larva.

The *Samia cynthia ricini* grown with the artificial diet for lepidopteran insects K25 was dissected and the content of ¹⁵N in the epidermis and silk glands including fat bodies was measured with a mass spectrometer. The result is shown in Table 5. In the *Samia cynthia ricini* that continued to feed on the diet containing the same amount of stable isotope to that in the control diet for 12 days, the contents of ¹⁵N in the epidermis and silk glands were 58.2 atom% and 87.4 atom%, respectively, showing an extremely high content of stable isotope.
On the other hand, when the *Samia cynthia ricini* grown with the artificial diet for lepidopteran insects KS40 or KM25 was dissected and the contents of ¹⁵N in the epidermis and silk glands including fat bodies were measured with a mass spectrometer, the contents of ¹⁵N detected were only the same level as that of the stable isotope of nitrogen found in nature.

**[Table 5]**

| Measurement results of ¹⁵N content in *Samia cynthia ricini* grown with artificial diet K25 for lepidopteran insects | |
|---|---|
| | ¹⁵N atom% |
| Epidermis of *Samia cynthia ricini* | 58.2 |
| Silk glands of *Samia cynthia ricini* | 87.4 |

### (Example 2)

As a protein added to the artificial diet for lepidopteran insects according to the present invention, a protein originating from the yeast strain *Saccharomyces cerevisiae* NBRC0203 was used. Cells of *Saccharomyces cerevisiae* were cultured for 22 hours in a culture medium having a composition shown in Table 6 which contained ¹⁵N labeled ammonium sulfate. After the cultivation, the cells of *Saccharomyces cerevisiae* in the culture medium were recovered by centrifugal separation. The collected cells were first washed with ion exchanged water and then frozen with liquid nitrogen, and thereafter freeze dried directly in a freeze dryer.

**[Table 6]**

| Composition of culture medium for labeling *S. cerevisiae* with ¹⁵N | |
|---|---|
| Substance | %(w/v) |
| D-glucose | 0.5 |
| ¹⁵N labeled ammonium sulfate | 0.25 |
| Yeast nitrogen base without amino acid & ammonium sulfate (manufactured by Difco Laboratories) | 0.17 |

About 37.3 g of the ¹⁵N labeled *Saccharomyces cerevisiae* cells obtained by drying was fmely ground and all of the resultant was placed in a 2L eggplant shaped flask. Subsequently, 1L of an aqueous solution containing 10% trichloroacetic acid (TCA) was added to the flask, and by subjecting the resulting mixture to a reflux/extraction by heating for 1.5 hours due to the heating of the flask to 100°C using an oil bath, a deglycosylation treatment as well as an extraction/removal treatment of water-soluble, low molecular weight components were carried out. Thereafter, the resultant was cooled down to room temperature and the solution was then transferred to a 500 ml centrifugation tube and was centrifuged at 8,000 rpm for 30 minutes to remove the supernatant. Ethanol was then added to wash the obtained precipitates by suspending the precipitates therein. The resulting suspension was then centrifuged at 8,000 rpm for 30 minutes and the resulting precipitates were recovered. After repeating this operation twice, the resulting precipitates were dried in a constant temperature vacuum dryer for 2 days.

The dried extract from *Saccharomyces cerevisiae* was placed in a 2L eggplant shaped flask, and by first adding 1L of a mixed solvent of methanol and chloroform (2:1) to the flask, and then subjecting the mixture to a reflux/extraction by heating for 1.5 hours due to the heating of the flask to 90°C using an oil bath, a defatting treatment was carried out. Thereafter, the mixture was cooled down to room temperature and then filtered using a Teflon filter. About 17.2 g of ¹⁵N labeled crude protein fraction originating from yeast cells was obtained by washing the matter obtained due to the filtration process first with methanol and then with diethyl ether, and then dehydrating and drying the resultant.

The artificial diet for lepidopteran insects containing the abovementioned crude yeast protein fraction labeled with ¹⁵N (S25M5) was mixed so as to achieve the composition shown in Table 7, uniformly kneaded while being heated, and was then steamed and formed into a rectangular shape. On the other hand, unlabeled *Bombyx mori* and *Samia cynthia ricini* were fed with the artificial diets KS40 and KM25 for unlabeled insects shown in Table 2 of Example 1 as controls. In the artificial diet S25M5 for lepidopteran insects, the content of stable isotope (¹⁵N) was equal to or greater than 90 atom%.

**[Table 7]**

| Composition of artificial diet for lepidopteran insects | |
|---|---|
| | S25M5 |
| ¹⁵N labeled crude protein | 25.000 g |
| Mulberry leaf powder | 5.000 g |
| Soybean oil | 3.000 g |
| Glucose | 10.000 g |
| Agar | 10.000 g |
| Phytosterol | 0.300 g |
| Inorganic salt mixture | 4.200 g |
| Mixture containing vitamins | 1.000 g |
| Chloramphenicol | 0.015 g |
| Ascorbic acid | 2.000 g |
| Citric acid | 4.000 g |
| Cellulose | 35.285 g |
| Sorbic acid | 0.200 g |
| Powder (in total) | 100.000 g |
| Propionic acid | 0.75 ml |
| Water | 249.25 ml |
| Total | 350.00 |

In addition, compositions of the inorganic salt mixture and the mixture containing vitamins in Table 7 are shown in Table 8 and 9, respectively.

**[Table 8]**

| Inorganic salt mixture | |
|---|---|
| Substance | Content (g) |
| K₂HPO₄ | 450 |
| CaCO₃ | 200 |
| KCl | 100 |
| MgSO₄ | 60 |
| FePO₄·4H₂O | 20 |
| ZnCl₂ | 2 |
| Total | 832 |

**[Table 9]**

| Mixture containing vitamins | |
|---|---|
| Substance | Content (g) |
| Biotin | 0.02 |
| Folic acid | 0.02 |
| Thiamine - HCl | 0.20 |
| Riboflavin | 0.20 |
| Pyridoxine - HCl | 0.30 |
| Nicotinic acid | 1.00 |
| Ca - panthothenate | 1.50 |
| Choline - Cl | 15.00 |
| Inositol | 20.00 |
| Cellulose powder | 61.76 |
| Total | 100.00 |

The artificial diet S25M5 for lepidopteran insects was fed to fifth instar larvae of *Bombyx mori* (silkworm) and *Samia cynthia ricini*, types of lepidopteran insects. In addition, the artificial diets for lepidopteran insects KS40 and KM25 which were used as controls were also fed to the different fifth instar larvae of *Bombyx mori* and *Samia cynthia ricini* in a similar manner. It should be noted that the amount of ingested artificial diet for lepidopteran insects, regardless of either ¹⁵N labeled or unlabeled, was same in respective cases of larvae of *Bombyx mori* and *Samia cynthia ricini.* The feeding of the above larvae of *Bombyx mori* and *Samia cynthia ricini* was continued until they pupated.

In addition, the fifth instar larvae of *Bombyx mori* and *Samia cynthia ricini* immediately after the ecdysis were inoculated with a solution containing nucleopolyhedrovirus (50 µL for *Bombyx mori* and 100 µL for *Samia cynthia ricini*) using an injection syringe. Then the larvae were fed with the artificial diet for lepidopteran insects S25M5 labeled with ¹⁵N and were bred at 25°C in a similar manner to that described earlier. Immediately after the death due to the virus infection (5 days after the inoculation for *Bombyx mori* and 9 days after the inoculation for *Samia cynthia ricini*), the larvae were frozen in a freezer (-20°C) for storage. The frozen larvae of *Bombyx mori* and *Samia cynthia ricini* were ground in a phosphate buffer solution where a small amount of phenylthiourea was added and the resultant was filtered with 3 sheets of gauze. The resulting filtrate was subjected to a low speed centrifugation at 3,000 rpm for 15 minutes and the supernatant was then removed. Distilled water was added to the obtained precipitates and the precipitates were suspended therein and the resulting suspension was refrigerated (4°C) for a few days. The abovementioned suspension was subjected to a low speed centrifugation at 3,000 rpm for 15 minutes and the supernatant was then removed. The obtained precipitates were suspended in a Percoll solution (distilled water : Percoll = 3 : 5) and the suspension was subjected to an ultracentrifugation at 25,000 rpm for 30 minutes using a centrifugal separator SW27.5 manufactured by Hitachi High-Technologies Corporation. The supernatant obtained after the ultracentrifugation was removed and the precipitates containing crudely purified polyhedra were washed with water and then suspended in about 1 ml of distilled water. The polyhedra suspension was layered onto a 50% to 80% sucrose density gradient solution and the resultant was subjected to an ultracentrifugation at 20,000 rpm for 30 minutes. The band corresponding to polyhedra formed around 70% sucrose was recovered using an injection syringe and after preparing the 10 times dilution or higher dilution thereof with distilled water, the diluted solution was subjected to a low speed centrifugation at 3,000 rpm for 15 minutes and the supernatant was then removed. The obtained precipitates were further washed with water for several times and sucrose was removed sufficiently therefrom, and the purified polyhedra were recovered as the resulting precipitates.

The ¹⁵N contents in the slice of cocoon and in the purified nuclear polyhedra from the *Bombyx mori* and *Samia cynthia ricini* grown with the artificial diet for lepidopteran insects S25M5 labeled with ¹⁵N or the artificial diet K40 or KS25 served as a control were measured using a mass spectrometer. The results are shown in Tables 10 and 11.

**[Table 10]**

| Measurement results of ¹⁵N content in cocoons of *Bombyx mori* and *Samia cynthia ricini* grown with ¹⁵N labeled or unlabeled artificial diet for lepidopteran insects | | |
|---|---|---|
| Measurement sample | Number of rounds | ¹⁵N atom% |
| Cocoon of unlabeled *Bombyx mori* | 1 | 0.365 |
| | 2 | 0.365 |
| | 3 | 0.365 |
| Cocoon of ¹⁵N labeled *Bombyx mori* | 1 | 85.6 |
| | 2 | 84.4 |
| | 3 | 82.1 |
| Cocoon of unlabeled *Samia cynthia ricini* | 1 | 0.367 |
| | 2 | 0.368 |
| | 3 | 0.367 |
| Cocoon of ¹⁵N labeled *Samia cynthia ricini* | 1 | 90.3 |
| | 2 | 90.3 |
| | 3 | 90.2 |

**[Table 11]**

| Measurement results of ¹⁵N content in nuclear polyhedra purified from larvae of *Bombyx mori* and *Samia cynthia ricini* grown with ¹⁵N labeled or unlabeled artificial diet for lepidopteran insects and infected with virus | | |
|---|---|---|
| Measurement sample | Number of rounds | ¹⁵N atom% |
| Nuclear polyhedra originating from unlabeled *Bombyx mori* | 1 | 0.367 |
| | 2 | 0.368 |
| | 3 | 0.369 |
| Nuclear polyhedra originating from ¹⁵N labeled *Bombyx mori* | 1 | 83.5 |
| | 2 | 83.3 |
| | 3 | 83.3 |
| Nuclear polyhedra originating from unlabeled *Samia cynthia ricini* | 1 | 0.368 |
| | 2 | 0.368 |
| | 3 | 0.368 |
| Nuclear polyhedra originating from ¹⁵N labeled *Samia cynthia ricini* | 1 | 87.2 |
| | 2 | 87.2 |
| | 3 | 87.1 |

In the control experiment where the larvae were fed with unlabeled artificial diet, the contents of ¹⁵N in the cocoon samples and the nuclear polyhedra samples obtained from *Bombyx mori* and *Samia cynthia ricini* detected by mass spectrometry all showed the same level as that of the stable isotope of nitrogen found in nature. On the other hand, when the larvae were fed with the artificial diet for lepidopteran insects labeled with ¹⁵N, a high content of ¹⁵N was detected in all the samples including cocoons and nuclear polyhedra from *Bombyx mori* and *Samia cynthia ricini.*
In other words, the results showed that the silk yarn secreted by the lepidopteran insect grown with the artificial diet for lepidopteran insects labeled with ¹⁵N had extremely high ¹⁵N content. Moreover the results showed that when the lepidopteran insect labeled with ¹⁵N was infected with a nucleopolyhedrovirus, the virus propagated in the larval body and the nuclear polyhedra formed by incorporating the virus particles therein had a considerably high ¹⁵N content. This result suggests that it is possible to produce a recombinant protein labeled with a stable isotope by using an insect labeled with the stable isotope as a host and infecting it with the nucleopolyhedrovirus.

### INDUSTRIAL APPLICABILITY

Since the biological materials produced according to the present invention are highly labeled with a stable isotope, their three dimensional structures can be analyzed by NMR. Moreover, their qualitative analysis as well as their quantitative analysis can be carried out by mass spectrometry. Therefore, the present invention is extremely useful in the biotechnology industry.

## Claims

1. An artificial diet for lepidopteran insects, comprising:
a protein; and
a carbohydrate, wherein
a content of a stable isotope is equal to or greater than 50 atom%.

2. The artificial diet for lepidopteran insects according to Claim 1, wherein the protein originates from a microorganism.

3. The artificial diet for lepidopteran insects according to Claim 2, wherein the microorganism is one, or two or more microorganisms selected from the group consisting of algae, bacteria including photosynthetic bacteria, fungi, and yeasts.

4. The artificial diet for lepidopteran insects according to Claim 2, wherein the microorganism is subjected to a defatting treatment as well as an extraction treatment using a hydrothermal solution.

5. The artificial diet for lepidopteran insects according to Claim 1, further comprising an inorganic salt and a vitamin.

6. The artificial diet for lepidopteran insects according to Claim 1, wherein the stable isotope is one, or two or more stable isotopes selected from the group consisting of ¹³C, ¹⁵N, ²H, ¹⁷O, ¹⁸O, an ³³S.

7. A method for producing an artificial diet for lepidopteran insects, comprising:
a defatting treatment step of subjecting a microorganism to a defatting treatment;
a hydrothermal solution extraction treatment step of subjecting the microorganism to an extraction treatment using a hydrothermal solution; and
a mixing step of mixing a carbohydrate and a protein originated from the microorganism that is subjected to the defatting treatment step and the hydrothermal solution extraction treatment step.

8. A lepidopteran insect wherein a content of a stable isotope is equal to or greater than 50 atom%.

9. The lepidopteran insect according to Claim 8, which has been grown by feeding with an artificial diet for lepidopteran insects wherein
the artificial diet for lepidopteran insects includes a protein and a carbohydrate, in which a content of a stable isotope is equal to or greater than 50 atom%.

10. The lepidopteran insect according to Claim 8, which is infected with a genetically engineered baculovirus.

11. A method for producing a lepidopteran insect, comprising growing the lepidopteran insect by feeding with the artificial diet for lepidopteran insects of Claim 1.

12. The method for producing a lepidopteran insect according to Claim 11, further comprising subjecting the lepidopteran insect to an infection treatment with a genetically engineered baculovirus.

13. A biological material produced by the lepidopteran insect according to Claim 8.
